(19) Europäisches Patentamt
European Patent Office
Office européen des brevets

(11) **EP 1 743 675 A1**

(12) **DEMANDE DE BREVET EUROPEEN**

(43) Date de publication:
**17.01.2007 Bulletin 2007/03**

(51) Int Cl.:
***A61N 1/37*** *(2006.01)*

(21) Numéro de dépôt: **06291141.7**

(22) Date de dépôt: **12.07.2006**

| | |
|---|---|
| (84) Etats contractants désignés:<br>**AT BE BG CH CY CZ DE DK EE ES FI FR GB GR HU IE IS IT LI LT LU LV MC NL PL PT RO SE SI SK TR**<br>Etats d'extension désignés:<br>**AL BA HR MK YU**<br><br>(30) Priorité: **12.07.2005 FR 0507441**<br><br>(71) Demandeur: **ELA MEDICAL**<br>**92541 Montrouge (FR)** | (72) Inventeur: **Casset, Cyrille**<br>**91130 Ris Orangis (FR)**<br><br>(74) Mandataire: **Dupuis-Latour, Dominique et al**<br>**SEP Bardehle Pagenberg Dost Altenburg Geissler**<br>**14 boulevard Malesherbes**<br>**75008 Paris (FR)** |

(54) **Dispositif medical implantable actif de type prothèse cardiaque comprenant des moyens perfectionnes de discrimination entre stimulations auriculaires efficaces et non efficaces**

(57)  Ce dispositif comporte des moyens de délivrance d'impulsions de stimulation auriculaire, des moyens de recueil d'un signal endocavitaire auriculaire, et des moyens de détection de capture auriculaire, aptes à reconnaître la présence d'une onde évoquée consécutive à l'application de l'impulsion de stimulation, ces moyens de détection comportant des moyens d'analyse des variations d'une dérivée seconde du signal recueilli. Les moyens de détection de capture auriculaire comprennent des moyens pour calculer une fonction intégrant, pendant la durée d'une fenêtre (F) de détection auriculaire post-stimulation auriculaire (StimA), la valeur absolue de la dérivée seconde (S") du signal recueilli (S), et des moyens de discrimination entre stimulations efficaces et non efficaces en réponse à la comparaison d'un paramètre caractéristique de ladite fonction avec un critère prédéterminé.

FIG_2

EP 1 743 675 A1

**Description**

L'invention concerne les "dispositifs médicaux implantables actifs" tels que définis par la directive 90/385/CEE du 20 juin 1990 du Conseil des communautés européennes, plus précisément les dispositifs stimulateurs cardiaques, défibrillateurs et/ou cardioverteurs permettant de délivrer au coeur des impulsions électriques de faible énergie pour le traitement des troubles du rythme cardiaque.

Tous ces dispositifs comportent des moyens de détection d'activité, c'est-à-dire de détection des dépolarisations spontanées du myocarde, ainsi que des moyens de stimulation de ce myocarde.

Après une stimulation, il est important de pouvoir recueillir l'"onde évoquée", c'est-à-dire l'onde de dépolarisation induite par la stimulation de la cavité considérée, afin de déterminer si cette stimulation a été efficace ou non, par exemple pour ajuster l'amplitude et/ou la largeur de l'impulsion de stimulation.

Dans le cas d'une stimulation atriale, la recherche de l'onde évoquée est rendue difficile du fait qu'elle présente alors une amplitude beaucoup plus faible que dans le cas du ventricule, et qu'elle est en outre beaucoup plus précoce. On comprendra que, dans ces conditions, il soit très difficile de détecter la présence d'une onde P évoquée, par exemple dans le cas d'un test de capture atriale.

Diverses techniques ont été proposées à cet effet, et l'on pourra se référer par exemple au EP-A-1 433 497 (ELA Medical) pour une description d'un circuit de détection des potentiels cardiaques évoqués consécutifs à une stimulation. Ce document propose en particulier une technique de discrimination entre stimulations efficaces et inefficaces basée sur l'analyse des extrema de la dérivée seconde du signal auriculaire recueilli.

Pour certaines configurations d'onde de dépolarisation, la dérivée seconde peut cependant présenter plusieurs extrema significatifs susceptibles de perturber l'analyse et de conduire à de faux diagnostics, positifs ou négatifs.

L'un des buts de l'invention est de proposer une nouvelle technique de détection de l'onde P évoquée, tenant compte du caractère à la fois très précoce et de faible amplitude de cette dernière et réduisant encore plus les risques de faux diagnostics, même pour des profils atypiques de l'onde évoquée.

Le dispositif de l'invention est du type général divulgué par le EP-A-1 433 497 précité, c'est-à-dire comportant des moyens de délivrance d'impulsions de stimulation auriculaire, des moyens de recueil d'un signal endocavitaire auriculaire, et des moyens de détection de capture auriculaire, aptes à reconnaître la présence d'une onde évoquée consécutive à l'application de l'impulsion de stimulation, ces moyens de détection comportant des moyens d'analyse des variations d'une dérivée seconde du signal recueilli.

De façon caractéristique de l'invention, les moyens de détection de capture auriculaire comprennent des moyens pour calculer une fonction intégrant, pendant la durée d'une fenêtre de détection auriculaire post-stimulation auriculaire, la valeur absolue de la dérivée seconde du signal recueilli, et des moyens de discrimination entre stimulations efficaces et non efficaces en réponse à la comparaison d'un paramètre caractéristique de ladite fonction avec un critère prédéterminé.

Ce paramètre caractéristique peut notamment être :

- la valeur finale atteinte par la fonction à la fin de la fenêtre de détection auriculaire post-stimulation auriculaire, et/ou
- la durée mise par la fonction pour atteindre un pourcentage prédéterminé de cette valeur finale, et/ou
- la pente de la fonction au début de la fenêtre de détection auriculaire post-stimulation auriculaire.

Enfin, le dispositif comprend avantageusement des moyens de recherche du seuil de capture opérant par dichotomie.

On va maintenant décrire un exemple de mise en oeuvre du dispositif de l'invention, en référence aux dessins annexés où les mêmes références numériques désignent d'une figure à l'autre des éléments identiques.

La figure 1 est un chronogramme montrant la variation du signal recueilli par le circuit de détection auriculaire, ainsi que la dérivée seconde de ce signal.

La figure 2 est un chronogramme illustrant la manière dont est déterminée la fonction selon l'invention assurant la discrimination entre stimulations efficaces et non efficaces.

La figure 3 est une représentation comparative de la caractéristique représentant l'évolution dans le temps de cette fonction dans deux cas de stimulation, efficace et non efficace.

En ce qui concerne ses aspects logiciels, l'invention peut être mise en oeuvre par une programmation appropriée du logiciel de commande d'un stimulateur connu, par exemple de type stimulateur cardiaque ou défibrillateur/cardioverteur, comprenant des moyens d'acquisition d'un signal fourni par des sondes endocavitaires et/ou un ou plusieurs capteurs implantés.

L'invention peut notamment être appliquée aux dispositifs implantables commercialisés par ELA Médical, Montrouge, France tels que les appareils *Symphony* et *ELA Rhapsody.*

Il s'agit de dispositifs à microprocesseur programmables comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail.

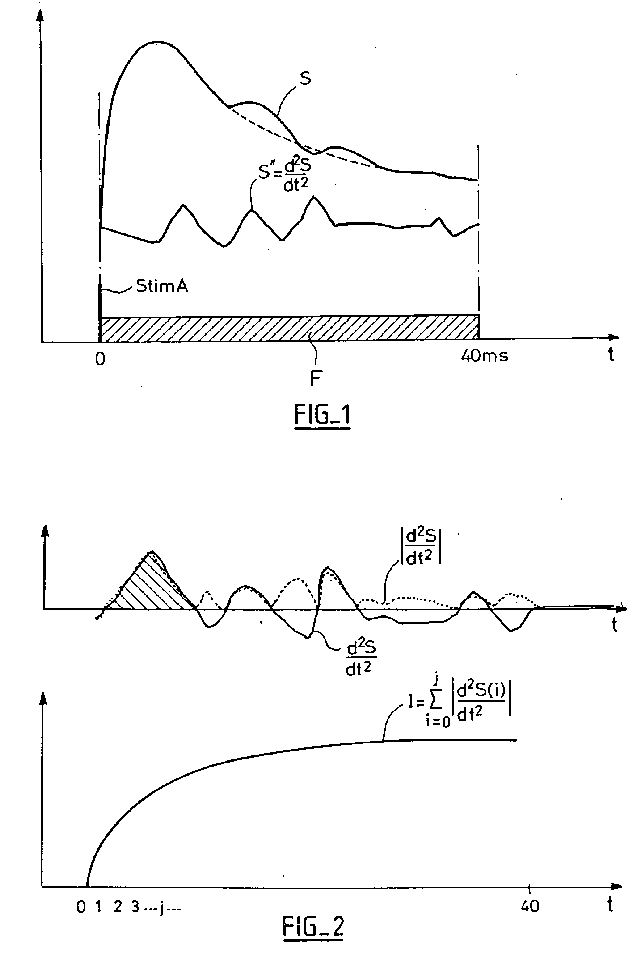

Sur la figure 1, la caractéristique S illustre la variation du signal EGM recueilli par les circuits de détection du dispositif implanté.

À la différence d'un signal ECG (de surface) où l'onde P est immédiatement reconnaissable, dans le cas d'un signal EGM (endocavitaire) le signal recueilli est un signal complexe, étalé dans le temps. Ce signal présente une phase d'augmentation rapide du potentiel d'une durée d'environ 10 ms, suivie d'une phase de décroissance lente s'étalant sur environ 30 ms. Cette phase de décroissance présente un profil légèrement accidenté dans le cas où un signal endocavitaire évoqué est effectivement présent, comme illustré en trait plein, et un profil moins accidenté dans le cas contraire, comme illustré en tiretés sur la figure.

C'est la discrimination entre ces deux profils qui permettra de déterminer s'il y a eu ou non capture, c'est-à-dire si la stimulation a été efficace ou non.

Cette discrimination est opérée en analysant le signal auriculaire endocavitaire S sur la durée d'une fenêtre de détection ou "fenêtre d'écoute" F, d'une durée fixe ou paramétrable de 40 ms par exemple, faisant suite à l'impulsion de stimulation auriculaire StimA (on fera abstraction de la période dite de "microblanking" immédiatement consécutive à la délivrance de l'impulsion de stimulation et pendant laquelle l'amplificateur est déconnecté pendant quelques dizaines de microsecondes pour éviter toute surcharge des circuits de détection).

A partir du signal endocavitaire auriculaire S(t) recueilli, le dispositif détermine la dérivée seconde S"(t) = $d^2S/dt^2$. Cette dérivée seconde est aisée à calculer à partir d'échantillons numérisés, de sorte que cette technique peut être mise en oeuvre de façon simple et en temps réel au sein du microcontrôleur du dispositif par un algorithme approprié.

De façon caractéristique de l'invention, au lieu d'analyser directement cette dérivée seconde S" (comme dans le cas du EP-A-1 433 497 précité qui prévoit d'analyser les extrema de la dérivée seconde), le dispositif calcule à partir de cette dérivée seconde S" une fonction 1(t) déterminée de la manière suivante.

Tout d'abord, comme illustré sur le chronogramme supérieur de la figure 2, le dispositif calcule, à partir de la dérivée seconde S"(t) = $d^2S/dt^2$ (en trait plein), la valeur absolue $|d^2S/dt^2|$ de cette dérivée seconde (en pointillés).

Ensuite, comme illustré sur le chronogramme du bas de la figure 2, cette valeur absolue est intégrée sur le temps pendant la durée de la fenêtre d'écoute F. Le signal étant constitué d'échantillons successifs numérisés S(1)... S(j)... S(40), cette étape d'intégration peut être réalisée en temps réel, pour chaque échantillon temporel j successif, par une simple opération de sommation de valeurs opérée par l'algorithme, en calculant l'expression suivante :

$$I(j) = \sum_{i=0}^{j} |d^2S(i)/dt^2|.$$

La fonction I se révèle en pratique très représentative du point de savoir si la capture a été efficace ou non.

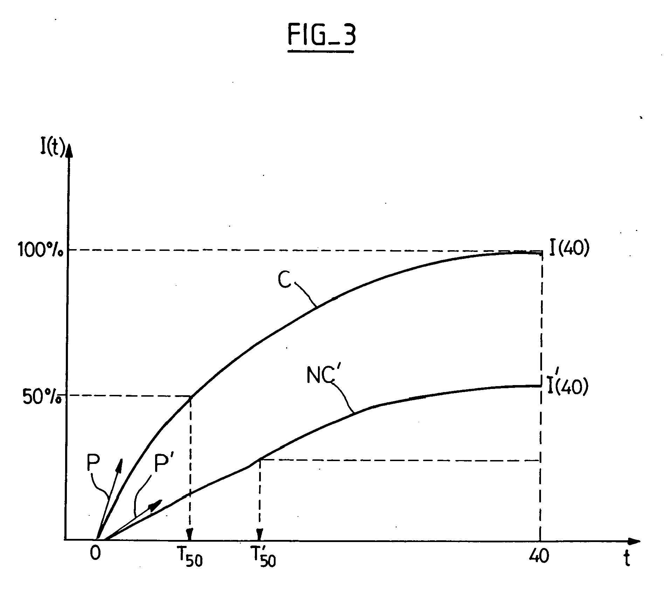

On a ainsi représenté sur la figure 3 deux exemples typiques de courbes I, l'une C correspondant à un cas de capture, l'autre NC correspondant à un cas d'absence de capture.

Cette fonction I est monotone (car elle intègre une valeur toujours positive), et les résultats montrent qu'elle continue à croître même en fin de fenêtre d'écoute, car la présence d'une onde P évoquée se manifeste pratiquement sur toute la durée de cette fenêtre.

Dans le cas où il y a capture, la fonction I croît plus rapidement, et atteint une valeur finale plus élevée.

La discrimination peut être effectuée en analysant un ou plusieurs paramètres remarquables de la caractéristique obtenue, notamment :

- la valeur finale (respectivement I(40) et l'(40)) atteinte à la fin de la fenêtre d'écoute, et/ou
- la durée (respectivement $T_{50}$ et $T'_{50}$) nécessaire pour atteindre 50% (par exemple) de cette valeur finale, et/ou
- la pente à l'origine (respectivement P et P'), donnée par les valeurs initiales de la fonction I.

La technique que l'on vient de décrire peut notamment être utilisée pour la recherche d'un seuil de capture par dichotomie.

La recherche par dichotomie se distingue de la méthode classique consistant à rechercher le seuil par des stimulations successives à énergie croissante depuis l'énergie maximale, jusqu'à détecter la perte de capture, comme décrit par exemple dans le EP-A-1 287 849 (ELA Medical). Cette méthode classique souffre de plusieurs limites : perte d'énergie du stimulateur, nombre élevé de cycles asynchrones et risque de saturation du signal évoqué en raison de la forte énergie appliquée sur les premières stimulations.

En procédant par dichotomie, on atteindra plus rapidement le seuil recherché et on réduira le nombre de stimulations spécifiques (c'est-à-dire celles qui sont seulement destinées à déterminer le seuil de capture), ceci avec une précision meilleure, donc une meilleure information du praticien assurant le suivi du patient lorsque celui-ci examinera les données stockées dans l'implant.

L'algorithme de recherche du seuil est le suivant.

On désignera par A1, A2, ... les stimulations auriculaires successives, opérées à différentes tensions de stimulation, et on notera Ai = OK ou Ai = NOK le résultat du test de capture, c'est-à-dire le point de savoir si l'impulsion appliquée à un niveau de tension donné a été efficace ou non, c'est-à-dire a été suivie, ou non, d'une onde P évoquée.

La première stimulation est effectuée au niveau d'énergie minimal, typiquement A1 = 0 V.

> Si A1 = OK, alors
>
>> fin de l'itération (car des dépolarisations auriculaires sponta-
>>
>> nées sont présentes), et
>>
>> augmentation de la fréquence stimulée jusqu'à :
>>
>>> A1 = NOK (perte de capture), ou
>>>
>>> fréquence maximale autorisée atteinte.
>
> Si A1 = NOK, alors stimulation A2 = 2 V,
>
>> si A2 = OK, alors stimulation A3 = 1 V,
>>
>>> si A3 = OK, alors stimulation A4 = 0,5 V,
>>>
>>> si A3 = NOK, alors A4 = 1,5 V et arrêt du test.
>>
>> si A2 = NOK, alors A3 = 4 V,
>>
>>> si A3 = OK, alors A4 = 3 V,
>>>
>>> si A3 = NOK, alors arrêt du test, le seuil de capture est
>>>
>>> supérieur à 4 V.

Et itération comme ci-dessus, jusqu'à détection du seuil de capture.

L'algorithme opère ainsi une double dichotomie : tout d'abord sur l'intervalle [0 volt, 2 volts] si A2 est efficace, sinon sur l'intervalle [0 volt, 5 volts] si A2 est inefficace. Si A2 est efficace, on peut ainsi poursuivre la recherche du seuil de capture sur un intervalle plus réduit, et aboutir beaucoup plus rapidement au résultat du test.

**Revendications**

1. Un dispositif médical implantable actif de type prothèse cardiaque de stimulation, resynchronisation, cardioversion et/ou défibrillation, comportant :

   - des moyens de délivrance d'impulsions de stimulation auriculaire,
   - des moyens de recueil d'un signal endocavitaire auriculaire, et
   - des moyens de détection de capture auriculaire, aptes à reconnaître la présence d'une onde évoquée consécutive à l'application de l'impulsion de stimulation, ces moyens de détection comportant des moyens d'analyse des variations d'une dérivée seconde du signal recueilli, dispositif **caractérisé en ce que** les moyens de détection de capture auriculaire comprennent :
   - des moyens pour calculer une fonction (I(t)) intégrant, pendant la durée d'une fenêtre (F) de détection auriculaire post-stimulation auriculaire (StimA), la valeur absolue de la dérivée seconde (S") du signal recueilli (S), et
   - des moyens de discrimination entre stimulations efficaces et non efficaces en réponse à la comparaison d'un paramètre caractéristique de ladite fonction avec un critère prédéterminé.

2. Le dispositif médical de la revendication 1, où ledit paramètre caractéristique est la valeur finale (I(40) ; I'(40)) atteinte par ladite fonction (I(t)) à la fin de la fenêtre de détection auriculaire post-stimulation auriculaire.

**3.** Le dispositif médical de la revendication 1, où ledit paramètre caractéristique est la durée ($T_{50}$ ; $T'_{50}$) mise par ladite fonction (I(t)) pour atteindre un pourcentage prédéterminé de la valeur finale (I(40) ; I'(40)) que cette fonction atteint à la fin de la fenêtre de détection auriculaire post-stimulation auriculaire.

**4.** Le dispositif médical de la revendication 1, où ledit paramètre caractéristique est la pente (P ; P') de ladite fonction (I(t)) au début de la fenêtre de détection auriculaire post-stimulation auriculaire.

**5.** Le dispositif médical de la revendication 1, comprenant en outre des moyens de recherche du seuil de capture par dichotomie.

$$S'' = \frac{d^2S}{dt^2}$$

StimA

F

0                    40ms          t

FIG_1

$$\left|\frac{d^2S}{dt^2}\right|$$

$$\frac{d^2S}{dt^2}$$

$$I = \sum_{i=0}^{j} \left|\frac{d^2S(i)}{dt^2}\right|$$

0 1 2 3 ⋯ j ⋯                    40      t

FIG_2

6

## FIG_3

**Office européen**
**des brevets**

## RAPPORT DE RECHERCHE EUROPEENNE

Numéro de la demande

EP 06 29 1141

### DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | US 5 443 485 A (HOUSWORTH ET AL) 22 août 1995 (1995-08-22) | 1,2 | INV. A61N1/37 |
| Y | | 1,2 | |
| A | * colonne 4, ligne 17 - colonne 8, ligne 12; figures 1-5 * ----- | 3-5 | |
| Y | US 6 163 724 A (HEMMING ET AL) 19 décembre 2000 (2000-12-19) | 1,2 | |
| A | * colonne 22, ligne 23-50; figures 4,17-18d * ----- | 3-5 | |
| A | US 5 431 693 A (SCHROEPPEL ET AL) 11 juillet 1995 (1995-07-11) * colonne 5, ligne 62 - colonne 7, ligne 20; figures 1-9 * ----- | 1-5 | |
| D,A | EP 1 433 497 A (ELA MEDICAL) 30 juin 2004 (2004-06-30) * colonne 3, ligne 23-33 * * colonne 8, ligne 8-38 * * figures 1,5A-B,6 * ----- | 1-5 | **DOMAINES TECHNIQUES RECHERCHES (IPC)** A61N |
| A | US 5 697 957 A (NOREN ET AL) 16 décembre 1997 (1997-12-16) * colonne 10, ligne 60 - colonne 12, ligne 24; figures 35-50 * ----- | 1-5 | |
| A | US 4 766 900 A (CALLAGHAN ET AL) 30 août 1988 (1988-08-30) * colonne 4, ligne 31 - colonne 6, ligne 42; figures 3,5,6 * ----- | 1-5 | |

Le présent rapport a été établi pour toutes les revendications

| Lieu de la recherche | Date d'achèvement de la recherche | Examinateur |
|---|---|---|
| Munich | 2 novembre 2006 | Fischer, Olivier |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.......................................................
& : membre de la même famille, document correspondant

EPO FORM 1503 03.82 (P04C02)

## ANNEXE AU RAPPORT DE RECHERCHE EUROPEENNE
## RELATIF A LA DEMANDE DE BREVET EUROPEEN NO.

EP 06 29 1141

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

02-11-2006

| Document brevet cité au rapport de recherche | | Date de publication | Membre(s) de la famille de brevet(s) | | Date de publication |
|---|---|---|---|---|---|
| US 5443485 | A | 22-08-1995 | CA | 2170614 A1 | 16-03-1995 |
| | | | DE | 69403340 D1 | 26-06-1997 |
| | | | DE | 69403340 T2 | 11-12-1997 |
| | | | EP | 0717646 A1 | 26-06-1996 |
| | | | ES | 2104417 T3 | 01-10-1997 |
| | | | JP | 9502370 T | 11-03-1997 |
| | | | WO | 9507114 A2 | 16-03-1995 |
| US 6163724 | A | 19-12-2000 | AUCUN | | |
| US 5431693 | A | 11-07-1995 | CA | 2178485 A1 | 15-06-1995 |
| | | | DE | 69404672 D1 | 04-09-1997 |
| | | | DE | 69404672 T2 | 26-02-1998 |
| | | | EP | 0732961 A1 | 25-09-1996 |
| | | | ES | 2105882 T3 | 16-10-1997 |
| | | | JP | 9508819 T | 09-09-1997 |
| | | | WO | 9515785 A1 | 15-06-1995 |
| EP 1433497 | A | 30-06-2004 | AT | 324929 T | 15-06-2006 |
| | | | FR | 2849387 A1 | 02-07-2004 |
| | | | US | 2004167577 A1 | 26-08-2004 |
| US 5697957 | A | 16-12-1997 | DE | 69726356 D1 | 08-01-2004 |
| | | | DE | 69726356 T2 | 26-08-2004 |
| | | | EP | 0826392 A2 | 04-03-1998 |
| | | | JP | 10085344 A | 07-04-1998 |
| US 4766900 | A | 30-08-1988 | AUCUN | | |

EPO FORM P0460

EP 1 743 675 A1

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1433497 A
- EP 1287849 A